Europäisches Patentamt

(19) **European Patent Office**

Office européen des brevets

(11) Numéro de publication: **0 081 440 B1**

(12) **FASCICULE DE BREVET EUROPEEN**

(45) Date de publication du fascicule du brevet: **27.08.86**

(51) Int. Cl.⁴: **C 08 L 89/06,** A 61 K 37/12, A 61 L 17/00

(21) Numéro de dépôt: **82420159.4**

(22) Date de dépôt: **24.11.82**

(54) Procédé de préparation de formes nouvelles de collagène natif ou déréticulé, à structure hélicoidale préservée, associées à des mucopolysaccharides et leurs applications notamment dans les domaines cosmétologiques, pharmaceutiques, analytiques et autres.

(30) Priorité: **30.11.81 FR 8122691**

(43) Date de publication de la demande: **15.06.83 Bulletin 83/24**

(45) Mention de la délivrance du brevet: **27.08.86 Bulletin 86/35**

(84) Etats contractants désignés: **BE CH DE GB IT LI NL SE**

(56) Documents cités:
FR - A - 2 318 189
US - A - 3 527 225

CHEMICAL ABSTRACTS, vol. 92, 1980, page 179, no. 142023h, Columbus, Ohio, USA R.B. CUNDALL et al.: "Polyelectrolyte complexes. 2. Interaction between collagen and polyanions"
BIOCHEM. J., vol. 145, 1975, pages 601-605, G.B. R.A. GREENWALD et al.: "Interaction of cartilage proteoglycans with collagen-substituted agarose gels"
BIOCHEM. J., vol. 141, 1974, pages 445-454, G.B. R.A. GELMAN et al.: "Interactions of an intact proteoglycan and its fragments with basic homopolypeptides in dilute aqueous solution"
CHEMICAL ABSTRACTS, vol. 91, 1979, page 332, no. 216677c, Columbus, Ohio, USA

(73) Titulaire: **SOCIETE ANONYME DE DEVELOPPEMENT DES UTILISATIONS DU CUIR, Zone Indus. des Troques, F-69630 Chaponost (FR)**

(72) Inventeur: **Huc, Alain Roger, 96, chemin des Fonds, Sainte-Foy-lès-Lyon Rhône (FR)**
Inventeur: **Allard, Roland Louis, 53, rue du Général de Gaulle, Saint-Chamond Loire (FR)**
Inventeur: **Chavrier, Christian Louis, 10D, rue Claude Naudrant, Caluire Rhône (FR)**

(74) Mandataire: **Maureau, Pierre, Cabinet GERMAIN & MAUREAU Le Britannia - Tour C 20, Boulevard E. Déruelle, F-69003 Lyon (FR)**

## Description

La présente invention concerne un procédé de préparation de formes nouvelles de collagène, natif ou déréticulé, à structure hélicoïdale préservée, associées à des mucopolysaccharides, ainsi que les compositions obtenues par mise en œuvre dudit procédé et leurs applications, notamment dans les domaines cosmétologiques, pharmaceutiques, analytiques.

Les tissus conjonctifs, et la peau en particulier, sont constitués principalement de collagène. Cette protéine est donc responsable en grande partie de l'état de la peau. Au cours du vieillissement, le collagène se réticule – on dit qu'il se polymérise – et la peau se ride, tout en perdant son élasticité. L'apport de collagène jeune, à structure hélocoïdale préservée, peut pallier, du moins en partie, ce processus de vieillissement.

Par ailleurs, le derme de la peau renferme deux autres types de macromolécules, qui sont l'élastine et les protéoglycanes. Alors que la première remplit un rôle qui lui est propre en ne réagissant pas avec le collagène, les secondes peuvent avoir une influence sur les propriétés de la peau par l'intermédiaire de leurs interactions avec le collagène.

Les protéoglycanes sont des macromolécules constituées d'acide hyaluronique et de substances protéiques, sur lesquelles sont branchés les glycosaminoglycanes, polymères de masse moléculaire d'environ 50.000 constitués d'acide uronique et d'hexosamines que l'on appelle également mucopolysaccharides.

D'après «O. BRINK, COLSTON PAP., 26 (Struct. Fibrous Biopolym), 1975, 81–92», les interactions des protéoglycanes avec le collagène sont de deux types: exclusion stérique et liaisons électrostatiques. La première interaction est due à la taille importante des protéoglycanes et elle n'apparaît pratiquement pas dans le cas des glycosaminoglycanes. La deuxième interaction, par contre, due aux charges électrostatiques, existe aussi bien avec les protéoglycanes qu'avec les glycosaminoglycanes. D'autres auteurs ont également étudié le mécanisme de ces interactions (TC LAURENT, COLSTON PAP, 26 (Struct. Fibrous Biopolym.), 1975, 27–33), (A. HUC, P. MANNSCHOTT, D. HERBAGE et M. WEISS (1975) in Protides of Biological Fluids 22nd colloquium (H. PEETERS éd.) p. 91, Pergamon Press, OXFORD and NEW-YORK), (D. HERBAGE, J.M. LUCAS, A. HUC, Biochim. Acta, 336 (1974) 108–116). Certains auteurs ont mis en évidence leur influence sur les propriétés mécaniques des tissus (JACKSON et BENTLEY (1968) in Treatise on collagen, (GOULD B.S. éd.), vol. 2 A, p. 189, Academic Press, NEW-YORK). Enfin, les glycosaminoglycanes comportant de nombreux groupements chargés sont entourés d'une grande quantité d'eau, qui confère au tissu une partie de sa souplesse. L'action des glycosaminoglycanes sur la peau peut ainsi être résumée selon les trois critères suivants:

– Protection de la structure native du collagène,
– Influence sur les propriétés mécaniques des tissus,
– Hydratation de la peau.

On voit donc l'intérêt de l'association des glycosaminoglycanes avec le collagène pour de nombreuses applications.

On sait par ailleurs que le collagène a la propriété de passer partiellement en solution aqueuse, sous l'action d'agents organiques ou minéraux. Néanmois, sa solubilité reste faible et il n'est possible de mettre en solution qu'une fraction relativement faible de collagène, en général en milieu acide.

De nombreuses méthodes ont été proposées dans le but, non seulement d'extraire les fractions solubles du collagène, mais aussi d'augmenter la solubilité de la partie insoluble. Ces méthodes sont généralement basées sur le prétraitement des matières premières à l'aide d'agents chimiques ou d'enzymes plus ou moins spécifiques.

D'autres solutions préconisées consistent à utiliser par exemple l'action de la chaleur en milieu aqueux, mais dans ce cas, il se produit une dénaturation du collagène en gélatine, voire même une certaine hydrolyse du collagène, aboutissant à la rupture des liaisons transversales de la molécule et à la destruction de la structure hélicoïdale du collagène.

En règle générale, les fractions solubles du collagène en milieu aqueux ont des valeurs de pH inférieures ou égales à 4. Dans certains cas, cette acidité peut présenter des inconvénients majeurs pour l'utilisation du collagène soluble, dans la mesure où ces valeurs de pH sont inférieures en pH physiologique. Cela peut être le cas pour des applications cosmétiques, dermatologiques et même thérapeutiques.

Or, on sait que le collagène a un point isoélectrique voisin du pH physiologique et que la neutralisation de fractions solubles du collagène provoque généralement une précipitation. Seules de très faibles quantités peuvent rester solubilisées dans des solutions tampons, diminuant d'autant l'intérêt de leur utilisation.

De même, lorsque des mucopolysaccharides comme par exemple de l'acide chondroïtine-4-sulfate ou de l'acide chondroïtine-6-sulfate sont ajoutés en quantités assez importantes à une solution aqueuse de collagène natif, la protéine collagénique précipite, du fait des interactions physiques qui s'établissent entre les deux composés.

On a déjà proposé d'associer le collagène à des mucopolysaccharides pour certains traitements dermatologiques. C'est ainsi que le brevet français 2 362 632 propose d'incorporer à du sérum une certaine quantité de glycosaminoglycanes et de collagène, en même temps que des agents facilitant la lipolyse des tissus adipeux, mais aucune indication ne permet de réaliser cette association collagène/glycosaminoglycanes sous forme de solutions limpides ou de gels homogènes, toutes les proportions étant indiquées en «principe actif sec».

La demande de brevet japonais Kokai 7952733 (Chem. Abst. vol 91, 1977 page 332 – réf. 216677c) traite également de mélanges de ce type mais ne donne aucune indication sur l'homogénéité du mélange obtenu, qualité non prédominante en ce cas, en raison de la quantié importante d'excipient nécessaire permettant d'enrober le produit précipité dans une crème.

Cundall R.B. et al. (Int. J. Biol. Macromol. 1979, 1 (5) 215–22) (Chem. Abst. vol 92, 1980 p. 179, réf. 142023h) ont étudié les interactions électrostatiques prenant naissance entre des polyanions et des protéines, plus spécialement dans le cas de complexes formés à pH 3 entre du collagène et certains mucopolysaccharides et ont déterminé que ces complexes se dissociaient quand le pH était porté de 3 à 9.

Dans le J. Biomed. Mater. Res. 1979, 13 (5) 701–16 (Chem. abst., vol 91, 1979, p. 290, réf. 181406j) on a étudié la compatibilité dans le sang in vitro de mélanges collagènes/mucopolysaccharides, qui sont beaucoup plus hémocompatibles que le collagène pur; pour cette utilisation, il est précisé que le collagène est précipité par le chondroïtine-6-sulfate à bas pH.

Enfin, l'exemple 1 de l'U.S.P. 3 527 225 mentionne la possibilité d'obtention d'une solution d'un mélange de collagène et d'acide chondroïtine sulfurique; mais il faut noter que, dans les conditions d'expérimentation, il n'est pas possible de travailler avec une concentration en mucopolysaccharides supérieure à 3% par rapport à la concentration en collagène.

On a enfin proposé (brevet français 2 332 863) de réaliser la première couche d'une membrane multicouche destinée à la réalisation de peaux synthétiques à l'aide d'un composite réalisé par mise en contact intime de collagène et d'un mucopolysaccharide suivie d'une réticulation de l'ensemble. Il est toutefois précisé que le produit de réaction coprécipite dans le milieux aqueux dans lequel il se forme ce qui, bien évidemment, ne permet pas de isposer de solution limpides ou de gels homogènes.

La présente invention vise à pallier ces inconvénients en proposant un procédé qui permet de réaliser sous forme de solutions limpides ou de gels homogènes l'association des glycosaminoglycanes avec du collagène déréticulé ou natif tout en conservant la structure hélicoïdale et les propriétés physiques et chimiques dudit collagène.

C'est ainsi que l'invention a pour objet un procédé de préparation de compositions résultant de l'association de collagène et de mucopolysaccharides par mise en présence de collagène avec une solution de glycosaminoglycanes, le pH du mélange étant porté à 7. Il est caractérisé en ce que les réactifs sont utilisés sous forme dissoute, la concentration pondérale en collagène étant le double de la concentration en glycosaminoglycanes et en ce que l'association est obtenue sous forme de solution limpide.

L'invention a également pour objet un procédé de préparation de compositions résultant de l'association de collagène et de mucopolysaccharides par mise en présence de collagène avec une solution de glycosaminoglycanes, le pH du mélange étant porté à 7. Il est caractérisé en ce que le collagène est utilisé sous forme de suspension homogène de fibres déshydratées, la concentration pondérale en collagène étant le double de la concentration en glycosaminoglycanes et en ce que l'association est obtenue sous forme de gel homogène.

Selon un mode de réalisation, le collagène est tout d'abord déréticulé par contact prolongé du tissu collagénique avec une solution de soude à pH 14.

Le collagène utilisé peut être aussi du collagène natif.

L'invention concerne également les présentations sous forme de solutions, poudres, éponges, films, tubes et crins des compositions obtenues par mise en œuvre du procédé, ainsi que les applications de ces formes de présentation dans le domaine cosmétique, ainsi que dans les domaines pharmaceutiques, vétérinaires et thérapeutiques.

Dans ces domaines, les applications les plus intéressantes sont réalisées en paradontologie et en chirurgie osseuse.

Ces modes de présentation peuvent être aussi utilisés en tant que pansements hémostatiques et cicatriciels.

L'invention concerne également l'application des formes de présentation dans les domaines analytiques et spécialement en tant que supports de molécules actives et notamment d'enzymes.

La présente invention sera mieux comprise d'ailleurs et ses avantages ressortiront bien des exemples suivants de deux modes de réalisation qui l'illustrent sans toutefois la limiter.

Exemple 1
Préparation d'une solution limpide d'une composition à base de collagène déréticulé et de chondroïtine-4-sulfate.

a) Préparation de l'acide chondroïtine-4-sulfate à partir des cloisons nasales de bovins.

Les cloisons nasales fraîches sont tout d'abord lavées soigneusement dans une solution de chlorure de sodium 9 pour 1000. Elles sont ensuite hachées et broyées. Le broyat est alors placé à raison de 1 kg par litre dans une solution de potasse 0,5N. Après agitation, l'ensemble est laissé au repos à température ambiante pendant 24 h. Au bout de ce laps de temps, le surnageant est séparé des matières insolubles par centrifugation à 30.000 g pendant 50 min. De l'acide acétique pur est ajouté au surnageant pour neutraliser la soude. La solution est alors concentrée cinq fois par évaporation sous vide. Le concentrat est versé dans un volume trois fois supérieur d'éthanol. Le précipité récupéré par décantation est dissous dans l'eau permutée. L'acide chondroïtine-4-sulfate est obtenu par lyophilisation de cette solution.

b) Préparation du collagène déréticulé.
Des peaux de veaux provenant d'animaux fraîchement abattus sont tout d'abord lavées à l'eau du robinet par brassage pendant une heure dans un foulon.

Le système pileux et le tissu sous-cutané sont séparés du derme à l'aide d'une refendeuse à bande rotative. Le derme récupéré est haché et broyé. Le broyat est lavé par trois bains successifs de tampon phosphate (pH 7,8). Entre chaque bain, le broyat est séparé de la solution par centrifugation en continu à 4000 t/min. Le résidu est alors rincé par deux bains successifs d'eau permutée et le liquide est séparé du broyat de la même façon que dans les opérations précédentes. Ces premiers traitements servent à éliminer les substances non collagéniques. Le tissu est alors placé dans une cuve contenant une solution de soude à pH 14. Après agitation pendant une demi-heure environ, l'ensemble est laissé au repos pendant huit jours. Le milieu est ensuite acidifié par l'acide chlorhydrique jusqu'à obtention d'un pH de 2. Dans le mélange obtenu, du chlorure de sodium est ajouté jusqu'à obtention d'une concentration de 10%. Le collagène précipité est dialysé contre de l'eau permutée stérile.

c) Préparation de la solution limpide d'une composition selon l'invention.

Le gel de collagène dialysé a une concentration de 1,2%, est dilué à raison de deux fois par une solution contenant 0,6% de l'acide chondroïtine-4-sulfate préparé en a), additionnée de 0,4% d'un produit conservateur tel que celui commercialisé sous la dénomination Phénonip. On ajoute à ce mélange une solution de soude 0,1N jusqu'à ce que le pH du mélange soit égal à 7. A ce moment, on obtient une solution limpide dans laquelle la concentration en collagène est de 0,6% et celle de glycosaminoglycane de 0,4%.

La solution ainsi obtenue peut être incorporée par exemple dans une crème cosmétique.

D'autres applications cosmétiques (masques pour le visage), pharmaceutiques ou thérapeutiques (paradontologie, chirurgie osseuse) analytiques (support de molécules actives, et notamment d'enzymes) imposent toutefois de disposer de la composition selon l'invention sous forme de gel homogène pouvant être ensuite facilement transformé en formes facilement utilisables telles qu'éponges, comme décrit dans l'exemple 2.

Exemple 2

Préparation d'un gel homogène selon l'invention.

a) Préparation de l'acide chondroïtine-6-sulfate.

On prépare cet acide à partir d'ailerons de requins selon le processus décrit en a) dans l'exemple 1.

b) Préparation d'une suspension homogène de collagène.

On prépare une pâte de collagène à partir de peaux d'origine animale par broyage suivi de malaxage en présence d'acides organiques chlorés selon le processus décrit dans le brevet français n° 1 568 829 de la demanderesse. Cette pâte est diluée à l'eau de façon à obtenir une concentration de 1% en collagène. La protéine contenue dans la solution est précipitée par du chlorure de sodium à 10%. Les fibres obtenues sont séparées du surnageant par centrifugation en continu à 4000 t/min. Elles sont ensuite déshydratées par séjour dans trois bains successifs d'acétone. Le séchage des fibres est effectué par chauffage à 30 °C dans une étuve ventilée.

Ces fibres sont placées dans un bain d'eau permutée à une concentration de 2 g/l. Après agitation pendant une heure, un gel est obtenu.

c) Préparation d'un gel homogène de la composition selon l'invention.

On ajoute au gel un volume égal d'une solution à 1 g/l d'acide chondroïtine-6-sulfate préparé en a). Le gel a tendance à se casser et une dispersion est obtenue. Une solution de soude 0,1N est alors versée sous agitation dans le mélange jusqu'à obtention d'un pH égal à 7. La dispersion se transforme alors à nouveau en un gel homogène.

On peut ensuite transformer ce gel, par exemple en éponge. On place le gel dans un plateau en quantité telle que son épaisseur soit inférieure à 10 mm. Une opération de lyophilisation ultérieure permet d'obtenir une éponge sur le plateau.

Les éponges ainsi obtenues ayant une composition assez voisine du tissu de soutien de l'os favorisent l'ostéogénèse quand elles sont placées dans des cavités osseuses en facilitant la réhabitation de ces cavités par les ostéocytes. Elles trouvent donc des applications en paradontologie et en chirurgie osseuse.

**Revendications**

1. Procédé de préparation de compositions résultant de l'association de collagène et de mucopolysaccharides, par mise en présence de collagène avec une solution de glycosaminoglycanes, le pH du mélange étant porté à 7, caractérisé en ce que les réactifs sont utilisés sous forme dissoute, la concentration pondérale en collagène étant le double de la concentration en glycosaminoglycanes et en ce que l'association est obtenue sous forme de solution limpide.

2. Procédé de préparation de compositions résultant de l'association de collagène et de mucopolysaccharides par mise en présence de collagène avec une solution de glycosaminoglycanes, le pH du mélange étant porté à 7, caractérisé en ce que le collagène est utilisé sous forme de suspension homogène de fibres déshydratées, la concentration pondérale en collagène étant le double de la concentration en glycosaminoglycanes et en ce que l'association est obtenue sous forme de gel homogène.

3. Procédé selon la revendication 1, caractérisé en ce que le collagène est tout d'abord déréticulé par contact prolongé du tissu collagénique avec une solution de soude à pH 4.

4. Procédé selon la revendication 2, caractérisé en ce que le collagène utilisé est du collagène natif.

5. Présentation des compositions obtenues par mise en œuvre du procédé selon l'une quelconque des revendications 1 à 4 sous forme de solutions, poudres, éponges, films, tubes et crins.

6. Application des formes de présentation selon la revendication 5 dans le domaine cosmétique.

7. Application des formes de présentation selon la revendication 5 dans les domaines pharmaceutiques, vétérinaires et thérapeutiques.

8. Application des formes de présentation selon la revendication 7 en paradontologie et chirurgie osseuse.

9. Application des formes de présentation selon la revendication 7 en tant que pansements hémostatiques et cicatriciels.

10. Application des formes de présentation selon la revendication 5 dans les domaines analytiques.

11. Application des formes de présentation selon la revendication 10 en tant que supports de molécules actives.

## Claims

1. A process for the preparation of compounds resulting from the association of collagen with mucopolysaccharides, by bringing together collagen and a solution of glycosaminoglycans, the pH of the mixture being adjusted to 7, characterized in that the reagents are used in dissolved form, the concentration by weight of collagen being double the concentration of glycosaminoglycans, and in that the association is obtained in the form of a clear solution.

2. A process for the preparation of compounds resulting from the association of collagen with mucopolysaccharides, by bringing together collagen and a solution of glycosaminoglycans, the pH of the mixture being adjusted to 7, characterized in that the collagen is used in the form of a homogeneous suspension of dehydrated fibres, the concentration by weight of collagen being double the concentration of glycosaminoglycans, and in that the association is obtained in the form of a homogeneous gel.

3. A process according to Claim 1, characterized in that the collagen is initially dereticulated by prolonged contact of collagenic tissue with a soda solution of pH 14.

4. A process according to Claim 2, characterized in that the collagen used is native collagen.

5. A preparation of compounds obtained by putting into practice the process according to any of Claims 1 to 4, in the form of solutions, powders, spondes, films, tubes and hairs.

6. The application of forms of preparation according to Claim 5 for cosmetic purposes.

7. The application of forms of preparation according to Claim 5 for pharmaceutical, veterinary, or therapeutic purposes.

8. The application of forms of preparation according to Claim 7 in paradontology and bone surgery.

9. The application of forms of preparation according to Claim 7 in the form of haemostatic and cicatricial bandages.

10. The application of forms of preparation according to Claim 5 for analytical purposes.

11. The application of forms of preparation according to Claim 10 in the form of supports for active molecules.

## Patentansprüche

1. Verfahren zur Herstellung von Zusammensetzungen, die aus der Assoziation von Kollagen und Mucopolysacchariden entstehen, durch das Zusammenbringen von Kollagen mit einer Lösung von Glykosaminoglycanen, wobei der pH der Mischung auf 7 gebracht wird, dadurch gekennzeichnet, dass die Reaktanten in gelöster Form eingesetzt werden, die Gewichtskonzentration des Kollagens das Doppelte derjenigen der Glykosaminoglycane beträgt und dass die Assoziation als klare Lösung erhalten wird.

2. Verfahren zur Herstellung von Zusammensetzungen, die aus der Assoziation von Kollagen und Mucopolysacchariden entstehen, durch das Zusammenbringen von Kollagen mit einer Lösung von Glykosaminoglycanen und wobei der pH auf 7 gebracht wird, dadurch gekennzeichnet, dass das Kollagen als homogene Suspension dehydratisierter Fasern verwendet wird, die Gewichtskonzentration des Kollagens das Doppelte der Konzentration von Glykosaminoglycanen beträgt und dass die Assoziation als homogenes Gel erhalten wird.

3. Verfahren nach Anspruch 1, dadurch gekennzeichnet, dass die Quervernetzungen des Kollagens zuallererst durch verlängerten Kontakt des kollagenhaltigen Gewebes mit einer Sodalösung bei pH 14 gespalten werden.

4. Verfahren nach Anspruch 2, dadurch gekennzeichnet, dass das verwendete Kollagen natives Kollagen ist.

5. Zusammensetzungen, die durch ein Verfahren nach einem der Ansprüche 1 bis 4 als Lösungen, Puder, Schwämme, Filme, Tuben und Haare/Fasern erhalten wurden.

6. Verwendung von Mitteln nach Anspruch 5 im kosmetischen Bereich.

7. Verwendung von Mitteln nach Anspruch 5 im pharmazeutischen, veterinären und therapeutischen Bereich.

8. Verwendung von Mitteln nach Anspruch 7 in der Paradontologie und Knochenchirurgie.

9. Verwendung von Mitteln nach Anspruch 7 als blutstillender und als Narbenverband.

10. Verwendung von Mitteln nach Anspruch 5 im analytischen Bereich.

11. Verwendung von Mitteln nach Anspruch 10 als Träger von aktiven Molekülen.